# FASCICULE DE BREVET EUROPEEN

(11) **EP 1 769 249 B1**
(45) Date de publication et mention de la délivrance du brevet: **02.09.2009**
(21) Numéro de dépôt: 05778861.4
(22) Date de dépôt: 21.06.2005
(51) Int. Cl.: G01N 33/569

(54) **NOUVEAUX POLYPEPTIDES POUR LA MISE EN ÉVIDENCE IN VITRO ET LA PRÉVENTION DES INFECTIONS À LEGIONELLA PNEUMOPHILA**
NEUE POLYPEPTIDE ZUR IN-VINTRO-ISOLIERUNG UND VORBEUGUNG VON INFEKTIONEN MIT LEGIONELLA PNEUMOPHILA
NOVEL POLYPEPTIDES FOR ISOLATING IN VITRO AND PREVENTING LEGIONELLA PNEUMOPHILA INFECTIONS

(30) Priorité: 29.06.2004 FR 0407114; 06.08.2004 FR 0408714; 08.12.2004 FR 0413063
(43) Date de publication de la demande: 04.04.2007
(73) Titulaire: InGen Biosciences, 91380 Chilly-Mazarin (FR); UNIVERSITE CLAUDE BERNARD - LYON 1, 69100 Villeurbanne (FR)
(72) Inventeur: ARIE, Jean-Philippe, F-75013 Paris (FR); CYNCYNATUS, Camille, F-75015 Paris (FR)
(74) Mandataire: Vial, Lionel
(86) Numéro de dépôt international: PCT/FR2005/001548
(87) Numéro de publication internationale: WO 2006/010805

(56) Documents cités:
- WO-A-87/06469
- WO-A-99/64610
- WO-A-20/05049642
- KWAIK YOUSEF ABU ET AL: "Phenotypic modulation by Legionella pneumophila upon infection of macrophages" INFECTION AND IMMUNITY, vol. 61, no. 4, 1993, pages 1320-1329, XP002438062 ISSN: 0019-9567
- CAZALET CHRISTEL ET AL: "Evidence in the Legionella pneumophila genome for exploitation of host cell functions and high genome plasticity" NATURE GENETICS, NEW YORK, NY, US, vol. 36, no. 11, novembre 2004 (2004-11), pages 1165-1173, XP002313851 ISSN: 1061-4036 -& DATABASE UniProt [Online] 23 novembre 2004 (2004-11-23), "Hypothetical protein." XP002438077 extrait de EBI accession no. UNIPROT:Q5X475 Database accession no. Q5X475
- DATABASE UniProt [Online] 8 novembre 2005 (2005-11-08), "10 kDa chaperonin (Protein Cpn10) (groES protein)." XP002438078 extrait de EBI accession no. UNIPROT:Q3J728 Database accession no. Q3J728
- BANGSBORG JETTE M ET AL: "Performance of four different indirect enzyme-linked immunosorbent assays (ELISAs) to detect specific IgG, IgA, and IgM in Legionnaires' disease" APMIS, vol. 102, no. 7, 1994, pages 501-508, XP008055172 ISSN: 0903-4641
- BLACK W J ET AL: "Legionella pneumophila zinc metalloprotease is structurally and functionally homologous to Pseudomonas aeruginosa elastase." JOURNAL OF BACTERIOLOGY. MAY 1990, vol. 172, no. 5, mai 1990 (1990-05), pages 2608-2613, XP002318264 ISSN: 0021-9193
- MOFFAT JENNIFER F ET AL: "Further molecular characterization of the cloned Legionella pneumophila zinc metalloprotease" INFECTION AND IMMUNITY, vol. 62, no. 2, 1994, pages 751-753, XP002318265 ISSN: 0019-9567
- MCINTYRE M ET AL: "RAPID IDENTIFICATION OF LEGIONELLA-PNEUMOPHILA ZINC METALLOPROTEASE USING CHROMOGENIC DETECTION" APMIS, vol. 99, no. 4, 1991, pages 316-320, XP008043164 ISSN: 0903-4641
- MALAN A K ET AL: "Comparison of two commercial enzyme-linked immunosorbent assays with an immunofluorescence assay for detection of Legionella pneumophila types 1 to 6" JOURNAL OF CLINICAL MICROBIOLOGY 01 JUL 2003 UNITED STATES, vol. 41, no. 7, 1 juillet 2003 (2003-07-01), pages 3060-3063, XP002318266 ISSN: 0095-1137

### LISTAGE DE SEQUENCES

<110> ABAG Université Claude Bernard Lyon 1
<120> Nouveaux polypeptides permettant la mise en évidence in vitro et la prévention des infections à Legionella pneumophila
<130> B3892
<150> 0407114
   <151> 2004-06-29
<150> 0408714
   <151> 2004-08-06
<150> 0413063
   <151> 2004-12-08
<160> 16
<170> PatentIn version 3.3
<210> 1
   <211> 1332
   <212> DNA
   <213> Legionella pneumophila
<220>
   <221> CDS
   <222> (1)..(1332)
<400> 1
   acg ttt gtt tta aag gaa ttt gat gca cta aaa agt cat ttt aac gac        48
   Thr Phe Val Leu Lys Glu Phe Asp Ala Leu Lys Ser His Phe Asn Asp
   1               5                   10                  15
   acc gtt aaa atc atc ctt caa cgc gaa aaa aag gac aaa att gaa gac        96
   Thr Val Lys Ile Ile Leu Gln Arg Glu Lys Lys Asp Lys Ile Glu Asp
               20                  25                  30
   ctt ccc aac ccc aga aaa gaa gag ctt caa ttt ctg acc gct gtt ctc       144
   Leu Pro Asn Pro Arg Lys Glu Glu Leu Gln Phe Leu Thr Ala Val Leu
           35                  40                  45
   aat caa ttg gaa gca aaa atc gat gaa ctg aaa cca cgt tct ttg gcc       192
   Asn Gln Leu Glu Ala Lys Ile Asp Glu Leu Lys Pro Arg Ser Leu Ala
       50                  55                  60
   agc tat gtc cat gta ttt tat ggt gct atg ctg ctt gtc tgt aaa gac       240
   Ser Tyr Val His Val Phe Tyr Gly Ala Met Leu Leu Val Cys Lys Asp
   65                  70                  75                  80
   gtt gaa aat aat ctg cgg gtg atg gaa aag aaa gaa aac agc ttg ctg       288
   Val Glu Asn Asn Leu Arg Val Met Glu Lys Lys Glu Asn Ser Leu Leu
                   85                  90                  95
   ttt act cgc tta atg gat ggt atg ggt att tct gat gaa aat ata cca       336
   Phe Thr Arg Leu Met Asp Gly Met Gly Ile Ser Asp Glu Asn Ile Pro
               100                 105                 110
   aca tct gag cag aac atc atg ttt tac aga gga tta aac aaa ttc tta      384
   Thr Ser Glu Gln Asn Ile Met Phe Tyr Arg Gly Leu Asn Lys Phe Leu
           115                 120                 125
   aat ttc att tat gaa agc aat gat tct cgt aaa ggc tta aaa aag gag      432
   Asn Phe Ile Tyr Glu Ser Asn Asp Ser Arg Lys Gly Leu Lys Lys Glu
       130                 135                 140
   cat ttc ctg caa gtc ctt tcg tta aaa aag ata tac tct tta gcc aaa      480
   His Phe Leu Gln Val Leu Ser Leu Lys Lys Ile Tyr Ser Leu Ala Lys
   145                 150                 155                 160
   tta agt tat gag cag gaa gag gca gct gaa aat aat gct ttg gca aaa      528
   Leu Ser Tyr Glu Gln Glu Glu Ala Ala Glu Asn Asn Ala Leu Ala Lys
                   165                 170                 175
   tta act gct gat gga aaa acc aaa gcc aat gcg aac agc ttc cat gtg      576
   Leu Thr Ala Asp Gly Lys Thr Lys Ala Asn Ala Asn Ser Phe His Val
               180                 185                 190
   gaa aaa cca atc gat tca tcc att gtt gag caa ttc aaa tcc tgg gat      624
   Glu Lys Pro Ile Asp Ser Ser Ile Val Glu Gln Phe Lys Ser Trp Asp
           195                 200                 205
   gaa atg aaa ggc gct ctt cat caa tta att ctc gat gaa ctt tct gat      672
   Glu Met Lys Gly Ala Leu His Gln Leu Ile Leu Asp Glu Leu Ser Asp
       210                 215                 220
   aag aac gtt gct aaa att tca gct tta agt caa gcc cgc tcc gca caa      720
   Lys Asn Val Ala Lys Ile Ser Ala Leu Ser Gln Ala Arg Ser Ala Gln
   225                 230                 235                 240
   ctg aaa ttt tta caa acg atg gca gaa caa cta gac aag atc cct aac      768
   Leu Lys Phe Leu Gln Thr Met Ala Glu Gln Leu Asp Lys Ile Pro Asn
                   245                 250                 255
   caa tct ctt gag ccg tct gag aaa atg gcc att ctt gct gga gca atg      816
   Gln Ser Leu Glu Pro Ser Glu Lys Met Ala Ile Leu Ala Gly Ala Met
               260                 265                 270
   tat atc gtt cgt ggc caa ata gcc cag gag tat gga aaa gat cca tta      864
   Tyr Ile Val Arg Gly Gln Ile Ala Gln Glu Tyr Gly Lys Asp Pro Leu
           275                 280                 285
   agc aac gat aaa atc agc gct act gtg att cat aca ggt tta agc acg      912
   Ser Asn Asp Lys Ile Ser Ala Thr Val Ile His Thr Gly Leu Ser Thr
       290                 295                 300
   ata ctt cat gcc aat gct gat tgc tgt gaa gac aag gaa gta ctg att      960
   Ile Leu His Ala Asn Ala Asp Cys Cys Glu Asp Lys Glu Val Leu Ile
   305                 310                 315                 320
   gct gct gcg aat aaa ttt att cgt cat atg gtt att gag cgt cct gaa     1008
   Ala Ala Ala Asn Lys Phe Ile Arg His Met Val Ile Glu Arg Pro Glu
                   325                 330                 335
   caa tca aat aaa aaa atc act aag gaa tcc gtt cga gaa aac aac atg     1056
   Gln Ser Asn Lys Lys Ile Thr Lys Glu Ser Val Arg Glu Asn Asn Met
               340                 345                 350
   ttt tcc gac atc gcc ggc ttc caa ttg atc tct gtc ttg acg tta ata      1104
   Phe Ser Asp Ile Ala Gly Phe Gln Leu Ile Ser Val Leu Thr Leu Ile
           355                 360                 365
   caa aac atg atc aaa aca tgt cgt act gat gcc att gaa gct tgt gtc      1152
   Gln Asn Met Ile Lys Thr Cys Arg Thr Asp Ala Ile Glu Ala Cys Val
       370                 375                 380
   acc aag cgt aag gaa gaa ctc gaa gca tta aaa ccc aaa aaa gag ggt      1200
   Thr Lys Arg Lys Glu Glu Leu Glu Ala Leu Lys Pro Lys Lys Glu Gly
   385                 390                 395                 400
   tat tcc att gcg agt tca gtc act gga tat gta ggc agc tgg ttt aaa      1248
   Tyr Ser Ile Ala Ser Ser Val Thr Gly Tyr Val Gly Ser Trp Phe Lys
                   405                 410                 415
   aaa gca cca agc atg tct gaa gaa gac gaa gaa gat gac tta aaa gat      1296
   Lys Ala Pro Ser Met Ser Glu Glu Asp Glu Glu Asp Asp Leu Lys Asp
               420                 425                 430
   caa aac aca gca gaa gag acc agc aaa ccg acc gta                      1332
   Gln Asn Thr Ala Glu Glu Thr Ser Lys Pro Thr Val
           435                 440
<210> 2
   <211> 444
   <212> PRT
   <213> Legionella pneumophila
<400> 2
   Thr Phe Val Leu Lys Glu Phe Asp Ala Leu Lys Ser His Phe Asn Asp
   1               5                   10                  15
   Thr Val Lys Ile Ile Leu Gln Arg Glu Lys Lys Asp Lys Ile Glu Asp
               20                  25                  30
   Leu Pro Asn Pro Arg Lys Glu Glu Leu Gln Phe Leu Thr Ala Val Leu
           35                  40                  45
   Asn Gln Leu Glu Ala Lys Ile Asp Glu Leu Lys Pro Arg Ser Leu Ala
       50                  55                  60
   Ser Tyr Val His Val Phe Tyr Gly Ala Met Leu Leu Val Cys Lys Asp
   65                  70                  75                  80
   Val Glu Asn Asn Leu Arg Val Met Glu Lys Lys Glu Asn Ser Leu Leu
                   85                  90                  95
   Phe Thr Arg Leu Met Asp Gly Met Gly Ile Ser Asp Glu Asn Ile Pro
               100                 105                 110
   Thr Ser Glu Gln Asn Ile Met Phe Tyr Arg Gly Leu Asn Lys Phe Leu
   115 120 125
   Asn Phe Ile Tyr Glu Ser Asn Asp Ser Arg Lys Gly Leu Lys Lys Glu
   130 135 140
   His Phe Leu Gln Val Leu Ser Leu Lys Lys Ile Tyr Ser Leu Ala Lys
   145 150 155 160
   Leu Ser Tyr Glu Gln Glu Glu Ala Ala Glu Asn Asn Ala Leu Ala Lys
   165 170 175
   Leu Thr Ala Asp Gly Lys Thr Lys Ala Asn Ala Asn Ser Phe His Val
   180 185 190
   Glu Lys Pro Ile Asp Ser Ser Ile Val Glu Gln Phe Lys Ser Trp Asp
   195 200 205
   Glu Met Lys Gly Ala Leu His Gln Leu Ile Leu Asp Glu Leu Ser Asp
   210 215 220
   Lys Asn Val Ala Lys Ile Ser Ala Leu Ser Gln Ala Arg Ser Ala Gln
   225 230 235 240
   Leu Lys Phe Leu Gln Thr Met Ala Glu Gln Leu Asp Lys Ile Pro Asn
   245 250 255
   Gln Ser Leu Glu Pro Ser Glu Lys Met Ala Ile Leu Ala Gly Ala Met
   260 265 270
   Tyr Ile Val Arg Gly Gln Ile Ala Gln Glu Tyr Gly Lys Asp Pro Leu
   275 280 285
   Ser Asn Asp Lys Ile Ser Ala Thr Val Ile His Thr Gly Leu Ser Thr
   290 295 300
   Ile Leu His Ala Asn Ala Asp Cys Cys Glu Asp Lys Glu Val Leu Ile
   305 310 315 320
   Ala Ala Ala Asn Lys Phe Ile Arg His Met Val Ile Glu Arg Pro Glu
   325 330 335
   Gln Ser Asn Lys Lys Ile Thr Lys Glu Ser Val Arg Glu Asn Asn Met
   340 345 350
   Phe Ser Asp Ile Ala Gly Phe Gln Leu Ile Ser Val Leu Thr Leu Ile
           355                 360                 365
   Gln Asn Met Ile Lys Thr Cys Arg Thr Asp Ala Ile Glu Ala Cys Val
       370                 375                 380
   Thr Lys Arg Lys Glu Glu Leu Glu Ala Leu Lys Pro Lys Lys Glu Gly
   385                 390                 395                 400
   Tyr Ser Ile Ala Ser Ser Val Thr Gly Tyr Val Gly Ser Trp Phe Lys
                   405                 410                 415
   Lys Ala Pro Ser Met Ser Glu Glu Asp Glu Glu Asp Asp Leu Lys Asp
               420                 425                 430
   Gln Asn Thr Ala Glu Glu Thr Ser Lys Pro Thr Val
           435                 440
<210> 3
   <211> 1008
   <212> DNA
   <213> Legionella pneumophila
<220>
   <221> CDS
   <222> (1)..(1008)
<400> 3 <210> 4
   <211> 336
   <212> PRT
   <213> Legionella pneumophila
<400> 4

## Description

La présente invention concerne un outil de diagnostic sérologique multiparamétrique des infections à *Legionella pneumophila.*

L'invention comprend l'identification de nouveaux polynucléotides et polypeptides, leur production, leur optimisation et leur utilisation, d'une part, dans le domaine du diagnostic des infections impliquant *Legionella pneumophila*.

Les légionelloses sont des infections respiratoires sévères résultant de l'inhalation d'aérosols contaminés par des bactéries à croissance intracellulaire du genre *Legionella,* et qui se manifestent sous forme sporadique ou épidémique. Ces infections surviennent aussi bien en ville (infections communautaires) qu'en milieu hospitalier (infections nosocomiales). L'espèce *Legionella pneumophila* est responsable de 95% de l'ensemble des cas de légionellose.

Les légionelloses se présentent principalement comme des infections pulmonaires aiguës. Elles se caractérisent par la sévérité du tableau clinique : après une incubation de 2 à 10 jours et un début pseudo-grippal, le malade présente une température élevée, un malaise général, des douleurs abdominales, voire des troubles psychiques. L'atteinte radiologique est souvent bilatérale. La mortalité associée, particulièrement élevée chez certaines personnes dites à risque (sujets âgés ou souffrant d'un cancer, d'une hémopathie), varie de 10 à 20% (13% des 835 cas recensés en France en 2002).

Il est essentiel de pouvoir poser précocement le diagnostic de légionellose du fait de l'enjeu thérapeutique : en effet, les légionelles sont insensibles aux bêta-lactamines - qui sont les principaux antibiotiques prescrits en cas de pneumopathie - et il faut avoir recours à un traitement spécifique reposant sur les macrolides (érythromycine) ou les fluoroquinolones.

A l'heure actuelle, le diagnostic est réalisé par trois méthodes générales : la mise en évidence des bactéries à partir de prélèvements broncho-pulmonaires (soit directement par immunofluorescence soit par culture sur milieux sélectifs) ; la détection d'antigènes urinaires spécifiques ; la mise en évidence d'une augmentation significative du titre des anticorps dans le sérum (sérologie). L'examen direct des prélèvements réalisé par microscopie à fluorescence se pratique, pour la majorité des réactifs commercialisés, à l'aide d'anticorps monoclonaux ou polyclonaux qui reconnaissent l'ensemble des sérogroupes connus de *L. pneumophila.* Le principal inconvénient de cette technique est sa faible sensibilité. Sa spécificité est également imparfaite (∼90%), du fait de réactions immunologiques croisées avec certaines bactéries comme *Pseudomonas aeruginosa, Bordetella pertussis* ou *Bacteroides fragilis*. La culture sur milieux spécifiques est la méthode de choix, notamment dans un but épidémiologique (typage des souches), mais donne des résultats tardifs puisque les légionelles cultivent en 3 à 4 jours, voire plus. La technique d'immunofluorescence indirecte (IFI) est la technique la plus employée pour le diagnostic sérologique (McDade JE, Shepard CC, Fraser DW, Tsai TR, Redus MA et Dowdle WR (1977), Legionnaires' disease : isolation of a bacterium and demonstration of its role in other respiratory disease, N Engl J Med. 297 (22): 1197-203). Environ 70% des diagnostics au niveau européen sont réalisés par cette méthode.

Les tests urinaires sont rapides et très spécifiques. Néanmoins, leur sensibilité est très variable, de 50-90% ; cette forte variabilité est liée non seulement aux caractéristiques du patient mais aussi au fait que les tests actuels permettent seulement la détection de *L. pneumophila* sérogroupe 1. De plus, la cinétique d'apparition et de disparition des antigènes de *Legionella* dans les urines est aussi très variable selon les patients. Un test positif ne peut pas signer une légionellose en phase aiguë et la rechute ou la récidive ne peuvent pas être évaluées par cette méthode. Enfin, en présence d'une recherche positive d'antigènes urinaires de légionelles, il reste nécessaire de réaliser en parallèle une culture de prélèvements respiratoires pour l'isolement de la bactérie dans un but épidémiologique.

Le sérodiagnostic est tout particulièrement utile chez les patients qui ne produisent pas d'expectoration et en cas d'enquête épidémiologique. Il repose sur la mise en évidence d'une augmentation significative du taux des anticorps entre un premier sérum et un sérum prélevé 4 à 6 semaines plus tard. Environ 30% des cas de légionellose sont identifiés par cette méthode à l'heure actuelle. Néanmoins, les antigènes utilisés dans les tests actuels sont constitués de simples extraits préparés directement à partir de culture de légionelles inactivées par la chaleur
ou après ensemencement des bactéries dans le sac vitellin d'oeufs embryonnés. Les anticorps détectés reconnaissent en majorité des déterminants du lipopolysaccharide (LPS), qui peuvent être proches de déterminants du LPS d'autres bactéries et provoquer des réactions croisées. De fait, des réactions de ce type ont été décrites avec d'autres bactéries à Gram négatif telles que *Pseudomonas sp., Bacteroides sp., Bordetella sp*. et certaines entérobactéries.

En résumé, les pratiques courantes répondent imparfaitement aux attentes médicales pour établir le diagnostic de légionellose. En particulier, les tests sérologiques actuels reposent sur des méthodes de détection anciennes, non automatisables, ou utilisent des antigènes non caractérisés ("soupes antigéniques"), dont la spécificité et la sensibilité sont peu satisfaisantes. L'utilisation de la sérologie souffre de très grandes variations selon les malades, et les tests basés sur la lecture d'un titre unique précoce possèdent des valeurs prédictives positives très faible de l'ordre de 10-15% (Jarraud S, Reyrolle M et Etienne J. dans Freney J, Renaud F, Hansen W, Bollet C, Précis de bactériologie clinique, ed. ESKA, Paris, 2000).

Il existe donc un très grand besoin dans le domaine du diagnostic sérologique afin de réaliser des tests rapides, peu coûteux et non invasifs, et qui pourraient également permettre un diagnostic plus précoce. Enfin, dans l'avenir, en cas de développement d'approches vaccinales visant à prévenir les infections à *Legionella pneumophila,* de tels tests seront indispensables pour détecter les échecs de vaccination.

Les inventeurs ont ainsi identifié de nouveaux polynucléotides et de nouveaux polypeptides dont les utilisations possibles sont décrites ci-après.

La présente invention a comme objectifs additionnels de résoudre les déficiences des tests sérologiques actuels et de permettre une analyse plus précise et plus complète grâce à la détection des différents isotypes d'anticorps spécifiques des polypeptides et, éventuellement, de diagnostiquer différents sérogroupes et espèces de légionelles.

### Définitions

Les définitions suivantes sont données afin de faciliter la compréhension de certains termes utilisés dans cette description.

On entend par "polynucléotide", un polyribonucléotide ou un polydésoxyribonucléotide qui peut être un ADN ou un ARN modifié ou non.

Le terme polynucléotide inclut, sans limitation, un ADN simple brin ou double brin, un ADN composé d'un mélange d'une ou plusieurs région(s) simple brin et d'une ou plusieurs région(s) double brin, un ADN qui est un mélange de régions simple brin, double brin et/ou triple brin, un ARN simple brin ou double brin, un ARN composé d'un mélange d'une ou plusieurs région(s) simple brin et d'une ou plusieurs région(s) double brin et les molécules hybrides comprenant un ADN et un ARN qui peuvent comprendre des régions simple brin, double brin et/ou triple brin ou un mélange de régions simple brin et double brin. Le terme polynucléotide peut aussi comprendre un ARN et/ou un ADN comprenant une ou plusieurs régions triple brin. Les brins dans de telles régions peuvent provenir de la même molécule ou de molécules différentes. Par conséquent, les ADN ou ARN avec des squelettes modifiés pour la stabilité ou d'autres raisons sont inclus dans le terme polynucléotides. On entend également par polynucléotide, les ADN et ARN contenant une ou plusieurs bases modifiées. On entend par base modifiée, par exemple, les bases inhabituelles telles que l'inosine. Le terme polynucléotide vise également les polynucléotides de forme modifiée chimiquement, enzymatiquement ou métaboliquement. Les polynucléotides comprennent également les polynucléotides courts tels que les oligonucléotides.

On entend par "polypeptide", un peptide, un oligopeptide, un oligomère ou une protéine comprenant au moins deux acides aminés joints l'un à l'autre par une liaison peptidique normale ou modifiée.

Le terme polypeptide comprend les chaînes courtes, appelées peptides, oligopeptides et oligomères, et les chaînes longues, appelées protéines.

Un polypeptide peut être composé d'acides aminés autres que les 20 acides aminés codés par les gènes humains. Un polypeptide peut également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.

Un polypeptide peut être ramifié suite à une ubiquitination ou être cyclique avec ou sans ramification. Ce type de modifications peut être le résultat de processus de post-traduction naturels ou synthétiques, qui sont bien connus de l'homme du métier.

On entend, par exemple, par modifications d'un polypeptide, l'acétylation, l'acylation, l'ADP-ribosylation, l'amidation, la fixation covalente de flavine, la fixation covalente d'un hème, la fixation covalente d'un nucléotide ou d'un dérivé nucléotidique, la fixation covalente d'un lipide ou d'un dérivé lipidique, la fixation covalente d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, l'oxydation, le processus protéolytique, la phosphorylation, la prénylation, la racémisation, la sénéloylation, la sulfatation, l'addition d'acides aminés telles que l'arginylation ou l'ubiquitination (PROTEINS STRUCTURE AND MOLEC ULAR PROPERTIES, 2nd Ed, T.E. Creighton, W.H. Freeman and Company, New York (1993*) and* Wold, F., Posttranslational Protein Modifications : Perspectives and Prospects, pgs 1-12 in POSTTRANSLATIONAL COVALENT MODIFICATION OF PROTEINS, B.C. Johnson, Ed, Academic Press, New York (1983*) ;* Seifter et al., Meth. Enzymol. 182: 626-646 (1990*) and* Rattan et al., Protein Synthesis: Posttranslational Modifications and Aging, Ann. N.Y. Acad Sci. 663:48-62 (1992)).

On entend par "pourcentage d'identité" entre deux séquences polynucléotidiques ou polypeptidiques le pourcentage de nucléotides ou d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons entre deux séquences polynucléotidiques ou polypeptidiques sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. Cette comparaison peut être réalisée grâce à un programme, par exemple le programme EMBOSS-Needle (alignement global Needleman-Wunsch) à l'aide de la matrice BLOSUM62/Open Gap 10.0 et Extension Penalty de 0, 5 (Needleman et Wunsch (1970). J. Mol. Biol. 48, 443-453 *et* Kruskal, J.B. (1983), An overview of seguence comparison, In D. Sankoff and J.B. Kruskal, (ed), Time warps, strind edits and macromolecules : the theory and practice of sequence comparison, pp. 1-44 Addison Wesley).

Le pourcentage d'identité est calculé en déterminant le nombre de positions identiques pour lesquelles le nucléotide ou l'acide aminé est identique entre les deux séquences, en divisant ce nombre de positions identiques par le nombre total de positions dans la fenêtre de comparaison et en multipliant le résultat obtenu par 100.

Un premier polynucléotide ayant, par exemple, une identité d'au moins 95% avec un second polynucléotide est donc un polynucléotide comportant, au plus, 5 nucléotides modifiés sur 100 nucléotides, par rapport à la séquence dudit second polynucléotide. En d'autres termes jusqu'à 5% des nucléotides dudit second polynucléotide peuvent être délétés ou substitués par un autre nucléotide, ou ledit premier polynucléotide peut comporter jusqu'à 5% de nucléotides en plus par rapport au nombre total de nucléotides du second polynucléotide. Ces modifications peuvent être positionnées aux extrémités 3' et/ou 5', ou à un endroit quelconque entre ces extrémités, en un seul ou plusieurs emplacements (Computational Molecular Biology, Lesk A.M., ed, Oxford University Press, New York, 1988*;* Biocomputing: Informatics and Genome Projects, Smith, D.W., ed, Academic Press, New York, 1993 *;* Computer Analysis of Sequence Data, Part I, Griffin, A.M., and Griffin, H.G., eds., Humana Press, New Jersey, 1994 *;* Sequence Analysis in Molecular Biology, von Heinje, G., Academic Press, 1987)*.*

Par analogie, un polypeptide ayant, par exemple, une identité d'au moins 95% avec un second polypeptide est un polypeptide comportant, au plus, 5 acides aminés modifiés sur 100 acides aminés, par rapport à la séquence dudit second polypeptide. En d'autres termes jusqu'à 5% des acides aminés dudit second polynucléotide peuvent être délétés ou substitués par d'autres acides aminés, ou ledit premier polypeptide peut comporter jusqu'à 5% d'acides aminés en plus par rapport au nombre total d'acides aminés du second polypeptide. Ces modifications peuvent être positionnées aux extrémités amino et/ou carboxy-terminales,
ou à un endroit quelconque entre ces positions terminales, en un seul ou plusieurs emplacements (Computational Molecular Biology, Lesk, A.M., ed, Oxford University Press, New York, 1988*;* Biocomputing: Informatics and Genome Projects, Smith, D.W., ed., Academic Press, New York, 1993 *;* Computer Analysis of Sequence Data, Part I, Griffin, A.M., and Griffin, H.G., eds., Humana Press, New Jersey, 1994 *;* Sequence Analysis in Molecular Biology, von Heinje, G., Academic Press, 1987).

On entend par "cellule hôte", une cellule qui a été transformée ou transfectée, ou est capable de transformation ou de transfection, par une séquence polynucléotidique exogène.

On entend par "milieu de culture", le milieu dans lequel on purifie le polypeptide de l'invention. Ce milieu peut être constitué par le milieu extracellulaire et/ou le lysat cellulaire. Des techniques bien connues de l'homme du métier permettent également à ce dernier de redonner la conformation active au polypeptide, si la conformation dudit polypeptide a été altérée lors de l'isolation ou de la purification.

On entend par "fonction", l'activité biologique d'un polypeptide ou d'un polynucléotide.

La fonction d'un polypeptide conforme à l'invention est celle d'un antigène de *Legionella pneumophila,* et la fonction d'un polynucléotide conforme à l'invention est celle de coder ce polypeptide. La fonction, selon l'invention, d'une combinaison d'antigènes est de permettre d'établir un diagnostic d'une infection à *L. pneumophila,* mais aussi de réaliser des suivis thérapeutiques et, enfin, de préciser s'il s'agit d'une infection active ou aiguë, par exemple, par l'utilisation de la détection de différents isotypes d'anticorps.

On entend par "antigène", tout composé qui, seul ou en association avec un adjuvant ou porteur, est capable d'induire une réponse immunitaire spécifique. Cette définition comprend également tout composé, présentant une analogie structurale avec ledit antigène, capable d'induire une réponse immunologique dirigée contre ledit antigène.

On entend par "analogie structurale" une analogie aussi bien de la structure primaire (séquence) que de la structure secondaire (éléments structuraux), ou que de la structure tertiaire (structure tridimensionnelle) ou que de la structure quaternaire (association de plusieurs polypeptides dans un même complexe (BIOCHEMISTRY, 4ème Ed, L. Stryer, New York 1995).

On entend par "variant" d'un polynucléotide dit initial ou d'un polypeptide dit initial, respectivement, un polynucléotide ou un polypeptide qui en diffère par au moins un nucléotide ou un acide aminé, mais qui garde les mêmes propriétés intrinsèques, c'est-à-dire la même fonction.

Une différence dans la séquence polynucléotidique du variant peut altérer ou non la séquence d'acides aminés du polypeptide qu'il code, par rapport à un polypeptide initial. Néanmoins, par définition, ces variants doivent conférer la même fonction que la séquence polynucléotidique initiale, par exemple, coder un polypeptide ayant une fonction antigénique.

Le polynucléotide ou le polypeptide variant diffère généralement du polynucléotide initial ou du polypeptide initial par une (ou plusieurs) substitution(s), addition(s), délétion(s), fusion(s) ou troncation(s) ou plusieurs de ces modifications, prises en combinaison. Un variant non-naturel d'un polynucléotide initial ou d'un polypeptide initial peut être obtenu, par exemple, par mutagenèse dirigée ou par synthèse directe.

On définit comme "séquence polynucléotidique complémentaire à la séquence polynucléotidique", un polynucléotide qui peut être hybridé avec cette séquence polynucléotidique dans des conditions stringentes.

On entend généralement, mais pas nécessairement, par "conditions stringentes" les conditions chimiques qui permettent une hybridation lorsque les séquences polynucléotidiques ont une identité d'au moins 80%.

Ces conditions peuvent être obtenues selon les méthodes bien connues de l'homme du métier.

On entend par "anticorps", les anticorps monoclonaux, polyclonaux, chimériques, simple chaîne ou humanisés, ainsi que les fragments Fab, incluant les produits d'un Fab ou d'une banque d'expression d'immunoglobulines. Il est aussi possible d'utiliser, à la place des anticorps, d'autres molécules immunospécifiques, par exemple des récepteurs de cellules T (TCR) comme récemment décrit *(*Li et al. 2005. Directed evolution of human T-cell receptors with picomolar affinities by phage display. Nat Biotechnol. 23:349-54*)* ou des molécules sélectionnées pour leur fixation spécifique, par exemple par évolution dirigée (voir, par exemple, Conrad et Scheller. 2005. Considerations on antibody-phage display methodology. Comb Chem High Throughput Screen. 8:117-26).

Un anticorps immunospécifique peut être obtenu par administration à un animal d'un polypeptide donné suivie d'une récupération des anticorps produits par ledit animal par extraction depuis ses fluides corporels. On peut également administrer à l'animal un variant dudit polypeptide, ou des cellules hôtes exprimant ce polypeptide.

Le terme "immunospécifique" appliqué au terme anticorps, vis-à-vis d'un polypeptide donné, signifie que l'anticorps possède une meilleure affinité pour ce polypeptide que pour d'autres polypeptides connus de l'art antérieur.

Par sérum "positif", on entend un sérum contenant des anticorps, produits à la suite d'une infection à *L. pneumophila,* identifiés par leur liaison avec les polypeptides (antigènes) de l'invention.

On entend par "sensibilité" la proportion de malades infectés, identifiés selon l'art antérieur et donnés positifs par le diagnostic selon l'invention.

On entend par "spécificité" la proportion de donneurs de sang, testés comme témoins, soumis au diagnostic selon l'invention et donnés négatifs par le diagnostic selon l'invention.

On entend par "amorces spécifiques" de courtes séquences nucléotidiques capables de s'hybrider de façon spécifique, grâce à la complémentarité des bases, sur le brin d'ADN ou sur son brin complémentaire ; ces amorces sont choisies par l'homme de l'art de façon à encadrer la séquence d'ADN et à l'amplifier spécifiquement.

On entend par "fixation spécifique" une interaction entre deux molécules ayant une affinité de l'ordre de 10⁶ M, 5.10⁶ M, 10⁷ M, 5.10⁷ M, 10⁸ M, 5.10⁸ M, 10⁹ M ou supérieure.

La présente invention répond aux objectifs qu'elle s'est fixés plus haut en apportant de nouveaux polynucléotides et de nouveaux polypeptides.

L'invention a pour objet l'utilisation, *in vitro,* de la protéine de séquence ID SEQ N°4 d'une partie ou de variants de cette protéine, de cellules hôtes comprenant des vecteurs incluant un polynucléotide codant pour cette protéine ou un variant de ladite protéine, dans la production d'anticorps et le domaine du diagnostic *in vitro* de *L. pneumophila.* Selon une forme d'exécution particulière de l'invention, celle-ci a pour objet l'utilisation, *in vitro*, de la protéine de séquence ID SEQ N°4, en combinaison avec la protéine de séquence ID SEQ N°2, dans la production d'anticorps et le domaine du diagnostic *in vitro* de *L. pneumophila.* L'invention a également pour objet un kit de diagnostic et une composition pharmaceutique.

### Utilisation des polypeptides

Certains polypeptides issus de *L. pneumophila* ne permettent pas de diagnostiquer une infection à cette bactérie, par exemple les protéines Pal ou FlaA de *Legionella pneumophila*. La Déposante a cependant identifié, de façon tout à fait inattendue, qu'un tel diagnostic était possible en utilisant d'autres polypeptides issus de *L. pneumophila* qu'elle a spécifiquement identifiés et, même, des fragments de polypeptides issus de *L. pneumophila.*

L'identification des polypeptides selon l'invention est le résultat d'un crible et d'études approfondies que les séquences issues des programmes de génomique de *L. pneumophila* ne laissaient pas envisager.

Ainsi, la présente invention a pour objet l'utilisation, dans la production d'anticorps et dans le domaine du diagnostic *in vitro* d'infections à *L. pneumophila,* d'un polypeptide comprenant la séquence d'acides aminés ID SEQ N°4 (appelée protéine G5), codée par la séquence polynucléotidique ID SEQ N°3, ou de l'un des autres polypeptides selon la revendication 1.

La présente invention a également pour objet l'utilisation, dans la production d'anticorps et dans le domaine du diagnostic *in vitro* d'infections à *L. pneumophila,* de la combinaison d'un polypeptide, tel que défini dans la revendication 1, avec le polypeptide comprenant la séquence d'acides aminés ID SEQ N°2 (appelée 2A1), codée par la séquence polynucléotidique ID SEQ N°1,
ou d'un autre polypeptide selon la revendication 2.

Le pourcentage d'identité indiqué dans les revendications précitées est d'au moins 80%, mais mieux il est d'au moins 90%.Ainsi, des polypeptides conformes à l'invention peuvent comprendre des variants ou des fragments de la séquence d'acides aminés ID SEQ N° 4, éventuellement en combinaison avec des variants ou des fragments de la séquence d'acides aminés ID SEQ N°2.

Les échantillons biologiques testés peuvent être du sang, de l'urine, de la salive, du liquide de ponction de sérologie (par exemple liquide céphalo-rachidien, liquide pleural ou liquide articulaire) ou l'un de leurs constituants (par exemple, le sérum).

Le diagnostic *in vitro* d'une infection à *L. pneumophila* est effectué, par exemple, par des tests classiques de réactions immunologiques, tels que ELISA ou Western Blot. Ces tests utilisent l'un au moins des polypeptides ou la combinaision de polypeptides selon l'invention qui vient se fixer, de manière spécifique, aux anticorps sériques contre *L. pneumophila* présents dans les échantillons biologiques.

Grâce aux polypeptides et combinaison de polypeptides définis ci-dessus, l'invention permet la détection d'anticorps naturellement produits lors d'infections à *L. pneumophila.*

L'invention vise, en outre, l'utilisation des polypeptides selon l'invention, pour détecter, périodiquement, *in vitro,* les anticorps dirigés contre *L. pneumophila* et ainsi suivre l'évolution de la pathologie et de l'effet d'un traitement appliqué à un patient.

### Utilisation de vecteurs d'expression et de cellules hôtes

La présente invention a aussi pour objet l'utilisation d'au moins un polypeptide, selon l'invention, préparé par culture d'une cellule hôte comprenant un vecteur recombinant ayant, inséré, un polynucléotide codant pour ledit polypeptide.

De nombreux systèmes d'expression peuvent être utilisés comme, par exemple, les chromosomes, les épisomes, les virus dérivés. Plus particulièrement, les vecteurs recombinants utilisés peuvent être dérivés de plasmides bactériens, de transposons, d'épisome de levure, d'éléments d'insertion, d'éléments chromosomiques de levures, de virus tels que les baculovirus, les *papillonna virus* comme SV40, les *vaccinia virus*, les adénovirus, les *fox pox virus*, les *pseudorabies virus*, les rétrovirus.

Ces vecteurs recombinants peuvent également être des dérivés de cosmides ou de phagemides. La séquence polynucléotidique peut être insérée dans le vecteur recombinant d'expression par les méthodes bien connues de l'homme du métier.

Le vecteur recombinant peut comprendre des séquences polynucléotidiques de contrôle de la régulation de l'expression du polynucléotide ainsi que des séquences polynucléotidiques permettant l'expression et la transcription d'un polynucléotide selon l'invention et la traduction d'un polypeptide selon l'invention, ces séquences étant choisies en fonction des cellules hôtes mises en oeuvre.

L'introduction du vecteur recombinant dans une cellule hôte peut être effectuée selon les méthodes bien connues de l'homme du métier telles que la transfection par phosphate de calcium, la transfection par lipides cationiques, l'électroporation, la transduction ou l'infection.

Les cellules hôtes peuvent être, par exemple, des cellules bactériennes, telles que des cellules de streptocoques, de staphylocoques, d'*Escherichia coli* ou de *Bacillus subtilis* ; des cellules de champignons, telles que des cellules de levure et des cellules d'*Aspergillus* ; des cellules de *Streptomyces* ; des cellules d'insectes, telles que des cellules de *Drosophilia S2* et de *Spodoptera Sf9* ; des cellules animales, telles que les cellules CHO, COS, HeLa, C127, BHK, HEK 293 ou encore des cellules végétales.

Le polypeptide peut être purifié à partir des cellules hôtes, selon les méthodes bien connues de l'homme du métier, telles que la précipitation à l'aide d'agents chaotropiques comme les sels, en particulier le sulfate d'ammonium, l'éthanol, l'acétone ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Utilisation des polynucléotides

La présente invention a également pour objet l'utilisation, dans la production d'anticorps et dans le diagnostic d'une infection à *L. pneumophila,* du polynucléotide comprenant la séquence polynucléotidique ID SEQ N°3, codant, , pour la protéine ID SEQ N°4, ou d'un autre polynucléotide selon la revendication 6 ou d'une combinaison d'au moins l'un de ces polynucléotides avec le polynucléotide de séquence ID SEQ N°1, codant pour la protéine de séquence ID SEQ N°2 ou d'un autre polynucléotide selon la revendication 7.

Le pourcentage d'identité indiqué dans les revendications précitées est d'au moins 80%, mais mieux il est d'au moins 90%.Ainsi, des polynucléotides conformes à l'invention peuvent comprendre des variants ou des fragments de la séquence polynucléotidique ID SEQ N°3, éventuellement en combinaison avec des variants ou des fragments de la séquence d'acides aminés ID SEQ N°1.

Les polynucléotides de l'invention peuvent être obtenus par les méthodes standard de synthèse d'ADN ou d'ARN.

Les polynucléotides conformes à l'invention peuvent également comprendre des séquences polynucléotidiques comme les séquences 5' et/ou 3' non-codantes, telles que, par exemple, des séquences transcrites, des séquences non-traduites, des séquences signal d'épissage, des séquences polyadénylées, des séquences de liaison avec des ribosomes
ou encore des séquences qui stabilisent l'ARNm.

### Partie expérimentale

### A) Protocoles d'obtention des antigènes

### A.1. Clonage de la séquence codant pour les polypeptides ID SEQ N°2 ou 4.

Les gènes codant pour les séquences des polypeptides, qui sont des antigènes, sont obtenus par amplification par PCR à partir de l'ADN génomique de la bactérie *Legionella pneumophila* (souche Philadelphia-1, ATCC 33152), en utilisant, respectivement, des amorces spécifiques des séquences ID SEQ N°1 et 3,.

Le fragment correspondant, ainsi amplifié, est cloné dans un vecteur selon des techniques classiques bien connues de l'homme du métier. Ce vecteur permet la production des protéines clonées sous contrôle d'un promoteur inductible par l'isopropyl-thiogalactoside (IPTG). Les protéines clonées correspondent aux polypeptides ID SEQ N°2 et 4.

### A.2. Expression des protéines

Une souche d'*Escherichia coli* est transformée par les vecteurs d'expression précédemment décrits. Les bactéries sélectionnées sont mises à cultiver une nuit à 30°C sous agitation dans 30 ml de milieu Luria Bertani (LB, J. Miller, "A short Course in Bacterial Genetics", Cold Spring Harbor Laboratory Press, 1992*)* contenant de l'ampicilline à une concentration finale de 100 µg/ml. Le lendemain, la culture est diluée au 1/50^{ème} dans un volume final de 1 litre de milieu LB additionné d'ampicilline à une concentration finale de 100 µg/ml qui est incubée à 30°C sous agitation. Lorsque la turbidité de la culture atteint une valeur d'absorbance à 600 nm (en abrégé, A600) d'environ 0,7, la production de la protéine est induite par l'isopropyl-thiogalactoside (en abrégé, IPTG) à une concentration finale de 0,1 mM. Les bactéries sont récoltées par centrifugation (10 minutes à 1400xg et à 4°C) lorsque la turbidité de la culture atteint une A600 d'environ 1,5.

### A.3. Purification des protéines

Après centrifugation, les cellules sont remises en suspension dans un tampon Tris-HCl 20 mM à pH 8,0 contenant du saccharose à 0,5 mM, puis traitées par du lysozyme (0,2 g/l) en présence d'acide éthylènediaminotétraacétique (EDTA) 12,5 mM, de DNase, RNase et PMSF. La suspension est incubée 30 minutes à 4°C puis centrifugée 10 minutes à 4°C à 15500xg. Le culot est congelé à -20°C pendant au moins une nuit.

Les protocoles de purification diffèrent pour chaque protéine en fonction de sa solubilité et de ses caractères physicochimiques propres. La purification de la protéine 2E1, qui ne fait pas partie de l'invention et qui correspond à la séquence d'acides aminés ID SEQ N°6, codée par la séquence polynucléotidique ID SEQ N°5, est détaillée ci-après, à titre d'exemple. Après décongélation, les bactéries sont reprises dans un tampon Mes [acide 2-(N-morpholino) éthane sulfonique] 25 mM à pH 6,0, puis soniquées 4 fois 20 secondes dans la glace. Après centrifugation à 15500xg à 4°C pendant 30 minutes, le surnageant est filtré sur membrane de porosité 0,22 µm. Le filtrat est alors déposé sur une colonne échangeuse de cation (par exemple, SP-Sépharose 12 ml, Amersham Biosciences). Après lavage de la colonne, la protéine est éluée par un gradient linéaire de 0 à 1 M de NaCl dans du tampon Mes 25 mM à pH 6,0 en 20 volumes, de colonne. Les fractions contenant la protéine sont rassemblées, dialysées sur la nuit contre du tampon Mes 25 mM à pH 6,0 puis redéposées sur cette même colonne. La protéine est ensuite éluée par un nouveau gradient de NaCl optimisé pour la protéine 2E1. De nouveau, les fractions contenant la protéine sont rassemblées et les protéines sont précipitées par du sulfate d'ammonium à une concentration finale de 0,6 g/l. La solution est laissée au moins une nuit à 4°C puis centrifugée 30 minutes à 20800xg. Le culot est alors repris dans un volume le plus faible possible (en général 300 µl de tampon Na₂HPO₄/NaH₂PO₄ 50 mM pH 8,0 contenant du NaCl 100 mM puis déposé sur une colonne de gel filtration, par exemple Superdex HR75-10/30, Amersham). Les fractions éluées contenant la protéine sont rassemblées et du glycérol est ajouté jusqu'à une concentration finale de 20%. La protéine purifiée est alors stockée à -20°C jusqu'à son utilisation dans les tests.

Les concentrations en protéine sont déterminées spectrophotométriquement à partir des coefficients d'absorption calculés par la méthode de Pace (Pace et al. 1995. How to measure and predict the molar absorption coefficient of a protein. Protein Science 4, 2411-2423). La pureté de la protéine est vérifiée par analyse par électrophorèse SDS-PAGE et par spectrométrie de masse.

### B) Test de diagnostic in vitro

Il a été utilisé des sérums provenant de patients ayant eu une infection documentée à *Legionella pneumophila* (collection du laboratoire). L'infection a pu être mise en évidence soit par l'isolement/culture des bactéries à partir de prélèvements broncho-pulmonaires, soit par une séroconversion, ou encore par un test urinaire positif.

Les sérums témoins correspondaient à des sérums de donneurs de sang (collection du laboratoire).

La fixation, sur les protéines recombinantes purifiées (obtenue comme décrit précédemment), des anticorps présents dans les sérums peut être évaluée soit par des tests en Western Blot soit par la technique ELISA.

### Exemple B1 : Protocole de test pour les polypeptides selon l'invention en ELISA

La fixation des anticorps présents dans les sérums a été évaluée par des tests ELISA. Les plaques ELISA sont laissées pendant une nuit à 4°C en présence de 0,5 µg d'antigène purifié dans du "tampon salin phosphate" (PBS). Après quatre lavages par du PBS contenant du polyoxyéthylène sorbitan (Tween) à 0,05%, les plaques sont saturées une heure à 37°C par du PBS-Tween contenant 5% de lait demi-écrémé (250 µl par puits). Quatre nouveaux lavages sont réalisés, puis 100 µl de chaque sérum positif, à la dilution adéquate dans du tampon PBS-Tween contenant 5% de lait demi-écrémé, sont ajoutés dans chaque puits. La plaque est ensuite laissée à 25°C pendant 30 minutes. Après quatre nouveaux lavages, des anticorps (secondaires) anti-immunoglobulines G, M, ou A ou simultanément anti-immunoglobulines G et/ou A et/ou M, humaines de chèvre marqués à la phosphatase alcaline (par exemple 170-6462, Biorad) sont ajoutés pendant 30 minutes à 25°C après avoir été dilués, selon le protocole du fournisseur, dans du tampon PBS-Tween contenant 5% de lait demi-écrémé. Quatre nouveaux lavages sont réalisés puis 100 µl de substrat (phosphate de p-nitrophényle), pNPP, par exemple A-3469, Sigma) sont ajoutés. L'absorbance à 405 nm de chacun des puits est mesurée après une incubation de 30 minutes à 37°C.

Sont considérés comme "positifs" en ELISA, les sérums identifiés par leur liaison avec les polypeptides (antigènes) de l'invention.

### Exemple B2 : Protocole de test pour les polypeptides selon l'invention en Western Blot

La fixation éventuelle des anticorps présents dans les sérums peut également être évaluée par des tests en Western Blot, par exemple, sur des extraits totaux de bactéries produisant les polypeptides ou sur les protéines purifiées.

Pour cela, la membrane de nitrocellulose sur laquelle les protéines de l'extrait sont transférées est saturée pendant 30 minutes à l'aide d'une solution de "tampon salin phosphate" (PBS) contenant 3% de lait demi-écrémé. Après trois lavages par du PBS contenant du polyoxyéthylène sorbitan (Tween) à 0,05%, la membrane est mise en présence du sérum testé à la dilution adéquate dans du tampon PBS contenant 3% de lait demi-écrémé, pendant 45 minutes. Après trois nouveaux lavages, des anticorps (secondaires) anti-immunoglobulines G, M ou A ou simultanément anti-immmunoglobulines G et/ou A et/ou M humaines de chèvre, marqués à la phosphatase alcaline (par exemple 170-6462, Biorad), dilués, selon le protocole du fournisseur, dans du tampon PBS contenant 3% de lait demi-écrémé, sont ajoutés puis laissés pendant 30 minutes. Trois nouveaux lavages sont réalisés puis du 5-bromo-4-chloro-3-indolyl-phosphate et du nitroblue tetrazolium sont ajoutés selon les indications du fournisseur jusqu'à l'obtention du résultat. Un résultat "positif" correspond à une précipitation du substrat sur la membrane à la position du polypeptide étudié.

### C) Résultats et interprétation

Des résultats types sont présentés dans les différents exemples ci-après.

### Exemple C1 : Résultats (ELISA) obtenus en utilisant des polypeptides selon l'invention pour la détection d'anticorps spécifiques dans les sérums

Le tableau 1 présente les résultats obtenus pour le polypeptide 2E1 (ID SEQ N°6) qui ne fait pas partie de l'invention et pour le polypeptide 2A1 (ID SEQ N°2) qui fait partie de l'invention lorsqu'il est combiné avec le polypeptide G5 (ID SEQ N° 4) avec des anticorps secondaires reconnaissant les immunoglobulines A présentes dans des sérums.

**Tableau 1 : Résultats (ELISA) obtenus en utilisant des anti-IgA comme anticorps secondaires**

| | Polypeptide testé | |
|---|---|---|
| | 2E1 | 2A1 |
| Proportion de sérums "positifs" parmi 49 sérums de malades infectés par *L. pneumophila* diagnostiqués selon l'art antérieur et soumis au diagnostic selon l'invention | 51,0% | 45,0% |
| Proportion de sérums "négatifs" parmi 96 sérums de donneurs de sang testés comme témoins | 97,0 % | 98,0% |

D'après le tableau, on constate que, par le test ELISA, on identifie *in vitro* des infections à *Legionella pneumophila.* Il est donc démontré, d'une part, l'existence chez l'homme d'une réponse anticorps significative (la probabilité associée à un test de χ² est inférieure à 0,05) vis-à-vis de la protéine 2E1 ou 2A1 au cours des infections à *Legionella pneumophila* et, d'autre part, que les protéines 2E1 ou 2A1 sont pertinentes pour le diagnostic sérologique de ce type d'infection. Les résultats exposés ci-dessus, à propos des protéines 2E1 et 2A1 sont également valables pour les autres polypeptides selon l'invention.

De plus l'utilisation des polypeptides selon l'invention permet, dans certains cas, l'identification de malades non détectés à ce stade de l'infection (mais confirmés dans des analyses ultérieures) par les techniques de sérologie de l'art antérieur.

### Exemple C2 : Résultats (ELISA) obtenus dans le cadre de la détection d'isotype d'anticorps dans les sérums

L'utilisation de certains polypeptides, par exemple 2A1 (ID SEQ N°2) (tableau 2), pour la détection d'immunoglobulines d'un isotype particulier est particulièrement pertinente.

**Tableau 2 : Résultats (ELISA) obtenus en utilisant 2A1 (ID SEQ N°2) comme polypeptide et en détectant les anticorps grâce à différents anticorps secondaires**

| Anticorps secondaire utilisés | IgA | IgG | IgG ou A |
|---|---|---|---|
| Proportion de sérums "positifs" parmi 49 sérums de malades infectés par *L. pneumophila* diagnostiqués selon l'art antérieur et soumis au diagnostic selon l'invention | 45,0% | 43,0% | 59,0% |
| Proportion de sérums "négatifs" parmi 96 sérums de donneurs de sang testés comme témoins | 98,0% | 98,0% | 96,0% |

Il en découle que les polypeptides peuvent être utilisés pour mettre en évidence la présence de certains isotypes d'anticorps lors d'infections à *L. pneumophila.*

De plus il existe une complémentarité des résultats donnés par les différents isotypes, des patients possédant des anticorps IgA contre les polypeptides selon l'invention ne possédant pas toujours des anticorps IgG contre ce même polypeptide. Par conséquent, l'utilisation de différents isotypes d'anticorps pour un même antigène, permet d'augmenter la sensibilité du diagnostic sérologique (tableau 2).

### Exemple C3 : combinaison de polypeptides pour le diagnostic in vitro des infections à L. pneumophila

Il existe une complémentarité des résultats donnés par les différents antigènes ; des patients possédant des anticorps contre l'un des polypeptides selon l'invention ne possédant pas toujours des anticorps contre l'autre polypeptide.

Ainsi, l'utilisation conjointe de plusieurs antigènes permet d'augmenter la sensibilité de la méthode (Tableau 3).

**Tableau 3 : Résultats (ELISA) obtenus en utilisant des combinaisons de différents polypeptides, pour la détection d'immunoglobulines de type IgA dans les sérums de patients**

| Polypeptides testés | 2E1 | 2A1 | 2E1 et 2A1 |
|---|---|---|---|
| Proportion de sérums "positifs" parmi 49 sérums de malades infectés par *L. pneumophila* diagnostiqués selon l'art antérieur et soumis au diagnostic selon l'invention | 51,0% | 45,0% | 70,0% |
| Proportion de sérums "négatifs" parmi 96 sérums de donneurs de sang testés comme témoins | 97,0 % | 98,0% | 95,0% |

Des résultats similaires, sans différences statistiquement significatives, ont été obtenus pour la combinaison du polypeptide G5 et du polypeptide 2A1.

### Exemple C4 : Utilisation des polypeptides selon l'invention pour le suivi thérapeutique

Chez des patients en convalescence, la constatation de la diminution ou de l'augmentation, au cours du temps, des valeurs ELISA obtenues en détectant les immunoglobulines (anticorps) et les isotypes d'anticorps présents dans les sérums, par leur fixation aux polypeptides selon l'invention, indique la guérison du patient ou l'évolution de la maladie. L'utilisation des polypeptides selon l'invention permet donc de réaliser des suivis thérapeutiques, c'est-à-dire (i) d'évaluer l'effet d'un traitement et (ii) de suivre l'évolution de l'infection chez un patient.

### D) Conclusions

Les résultats des tests précédents démontrent l'existence d'une réponse anticorps significative au cours des infections à *Legionella pneumophila* vis-à-vis des polypeptides de l'invention et la pertinence de ces polypeptides et de leurs associations/combinaisons pour le diagnostic sérologique de ce type d'infection. De même, la combinaison des résultats obtenus par la détection des différents isotypes d'anticorps dans les sérums, pour chacun des polypeptides, est elle aussi pertinente.

Ainsi, l'utilisation conjointe de plusieurs antigènes et/ou la détection de plusieurs isotypes d'anticorps permet en outre :
(i) d'augmenter la sensibilité de la méthode ;
(ii) de déterminer l'évolution de la pathologie (par l'utilisation de certains antigènes et d'isotypes d'anticorps) et ;
(iii) de réaliser des suivis thérapeutiques à moindre coût.

## Revendications

1. Utilisation pour détecter, *in vitro,* dans un échantillon biologique, la présence d'anticorps produits à la suite d'une infection à *Legionella pneumophila* :
a) du polypetide de séquence d'acides aminés ID SEQ N°4 (protéine G5), ou
b) d'une partie de la séquence d'acides aminés ID SEQ N°4 ayant la même fonction que ladite séquence ID SEQ N°4, ou
c) d'une séquence d'acides aminés ayant au moins 80% d'identité avec la séquence d'acides aminés ID SEQ N°4 et ayant la même fonction que ladite séquence ID SEQ N°4, ou
d) d'une séquence d'acides aminés ayant au moins 80% d'identité avec la séquence définie sous b) et ayant la même fonction que ladite séquence ID SEQ N°4, étant entendu que:
par "ayant la même fonction que ladite séquence ID SEQ N°4" on entend une fonction d'antigène de *Legionella pneumophila.*

2. Utilisation selon la revendication 1 mettant en oeuvre, de plus :
a) un polypeptide de séquence d'acides aminés ID SEQ N°2 (protéine 2A1), ou
b) un polypeptide comprenant une partie de la séquence d'acides aminés ID SEQ N°2 ayant la même fonction que ladite séquence ID SEQ N°2, ou
c) un polypeptide comprenant une séquence d'acides aminés ayant au moins 80% d'identité avec la séquence d'acides aminés ID SEQ N°2 et ayant la même fonction que ladite séquence ID SEQ N°2, ou
d) un polypeptide comprenant une séquence d'acides aminés ayant au moins 80% d'identité avec la séquence définie sous b), et ayant la même fonction que ladite séquence ID SEQ N°2, étant entendu que:
par "ayant la même fonction que ladite séquence ID SEQ N°2" on entend une fonction d'antigène de *Legionella pneumophila.*

3. Utilisation selon la revendication 1 ou 2,
**caractérisée en ce que** le ou les polypeptide(s) est(sont) préparé(s) par culture d'une cellule hôte comprenant un vecteur recombinant ayant, inséré, un polynucléotide codant pour ledit ou lesdits polypeptide(s) et par isolement dudit ou desdits polypeptide(s) du milieu de culture.

4. Utilisation selon la revendication 3, quand elle dépend de la revendication 1, **caractérisé en ce que** le polynucléotide est de séquence ID SEQ N°3 (polynucléotide codant pour la protéine G5).

5. Utilisation selon la revendication 3, quand elle dépend de la revendication 2, **caractérisée en ce que** le polynucléotide est de séquence ID SEQ N°1 (polynucléotide codant pour la protéine 2A1).

6. Utilisation pour détecter, *in vitro,* dans des échantillons biologiques, la présence d'anticorps produits à la suite d'une infection à *Legionella pneumophila* :
a) du polynucléotide de séquence ID SEQ N°3, ou
b) d'un polynucléotide comprenant une partie de la séquence ID SEQ N°3 ayant la même fonction que ladite séquence ID SEQ N°3, ou
c) d'un polynucléotide comprenant une séquence polynucléotidique ayant au moins 80% d'identité avec la séquence ID SEQ N°3 et ayant la même fonction que ladite séquence ID SEQ N°3, ou
d) d'un polynucléotide comprenant une séquence polynucléotidique ayant au moins 80% d'identité avec la séquence définie sous b), et ayant la même fonction que ladite séquence ID SEQ N°3, ou
e) d'une séquence polynucléotidique complémentaire à une des séquences polynucléotidiques définies sous a), b), c), ou d), étant entendu que:
par "ayant la même fonction que ladite séquence ID SEQ N°3" on entend une fonction de codage d'un polypeptide antigène de *Legionella pneumophila.*

7. Utilisation selon la revendication 6, mettant en oeuvre, de plus:
a) un polynucléotide de séquence ID SEQ N°1, ou
b) un polynucléotide comprenant une partie de la séquence ID SEQ N°1 ayant la même fonction que ladite séquence ID SEQ N°1, ou
c) un polynucléotide comprenant une séquence polynucléotidique ayant au moins 80% d'identité avec la séquence polynucléotidique ID SEQ N°1 et ayant la même fonction que ladite séquence ID SEQ N°1, ou
d) un polynucléotide comprenant une séquence polynucléotidique ayant au moins 80% d'identité avec la séquence définie sous b), et ayant la même fonction que ladite séquence ID SEQ N°1, ou
e) un polynucléotide comprenant une séquence polynucléotidique complémentaire à la séquence polynucléotidique définie sous a), b), c), ou d), étant entendu que:
par "ayant la même fonction que ladite séquence ID SEQ N°1" on entend une fonction de codage d'un polypeptide antigène de *Legionella pneumophila*.

## Claims

1. Use of:
a) the polypeptide of amino acid sequence ID SEQ No. 4 (protein G5), or
b) part of the amino acid sequence ID SEQ No. 4 having the same function as said sequence ID SEQ No. 4, or
c) an amino acid sequence having at least 80% identity with amino acid sequence ID SEQ No. 4 and having the same function as said sequence ID SEQ No. 4, or
d) an amino acid sequence having at least 80% identity with the sequence defined under b) and having the same function as said sequence ID SEQ No. 4, for the *in vitro* detection of the presence of antibodies produced following a *Legionella pneumophila* infection in a biological sample,
wherein:
"having the same function as said sequence ID SEQ No. 4" is understood as meaning a *Legionella pneumophila* antigen function.

2. Use according to claim 1, additionally employing:
a) a polypeptide of amino acid sequence ID SEQ No. 2 (protein 2A1), or
b) a polypeptide comprising part of amino acid sequence ID SEQ No. 2 having the same function as said sequence ID SEQ No. 2, or
c) a polypeptide comprising an amino acid sequence having at least 80% identity with amino acid sequence ID SEQ No. 2 and having the same function as said sequence ID SEQ No. 2, or
d) a polypeptide comprising an amino acid sequence having at least 80% identity with the sequence defined under b) and having the same function as said sequence ID SEQ No. 2,
wherein:
"having the same function as said sequence ID SEQ No. 2" is understood as meaning a *Legionella pneumophila* antigen function.

3. Use according to claim 1 or 2, **characterised in that** the polypeptide(s) is(are) prepared by culturing a host cell comprising a recombinant vector with an inserted polynucleotide coding for said polypeptide(s), and by isolating said polypeptide(s) from the culture medium.

4. Use according to claim 3, when it is dependent on claim 1, **characterised in that** the polynucleotide is of sequence ID SEQ No. 3 (polynucleotide coding for protein G5) .

5. Use according to claim 3, when it is dependent on claim 2, **characterised in that** the polynucleotide is of sequence ID SEQ No. 1 (polynucleotide coding for protein 2A1).

6. Use of:
a) the polynucleotide of sequence ID SEQ No.3, or
b) a polynucleotide comprising part of sequence ID SEQ No. 3 having the same function as said sequence ID SEQ No. 3, or
c) a polynucleotide comprising a polynucleotide sequence having at least 80% identity with sequence ID SEQ No. 3 and having the same function as said sequence ID SEQ No. 3, or
d) a polynucleotide comprising a polynucleotide sequence having at least 80% identity with the sequence defined under b) and having the same function as said sequence ID SEQ No. 3, or
e) a polynucleotide sequence complementary to one of the polynucleotide sequences defined under a), b), c) or d), for the *in vitro* detection of the presence of antibodies produced following a *Legionella pneumophila* infection in biological samples,
wherein:
"having the same function as said sequence ID SEQ No. 3" is understood as meaning a function coding for a *Legionella pneumophila* antigen polypeptide.

7. Use according to claim 6, additionally employing:
a) a polynucleotide of sequence ID SEQ No. 1, or
b) a polynucleotide comprising part of sequence ID SEQ No. 1 having the same function as said sequence ID SEQ No. 1, or
c) a polynucleotide comprising a polynucleotide sequence having at least 80% identity with polynucleotide sequence ID SEQ No. 1 and having the same function as said sequence ID SEQ No. 1, or
d) a polynucleotide comprising a polynucleotide sequence having at least 80% identity with the sequence defined under b) and having the same function as said sequence ID SEQ No. 1, or
e) a polynucleotide comprising a polynucleotide sequence complementary to the polynucleotide sequence defined under a), b), c) or d),
wherein:
"having the same function as said sequence ID SEQ No. 1" is understood as meaning a function coding for a *Legionella pneumophila* antigen polypeptide.

## Patentansprüche

1. Anwendung zur *In-vitro*-Detektion der Präsenz infolge einer *Legionella pneumophila*-Infektion in einer biologischen Probe erzeugter Antikörper:
a) des Polypeptids der Aminosäuresequenz SEQ ID NO:4, (G5-Protein), oder
b) eines Teils der Aminosäuresequenz SEQ ID NO:4 mit der gleichen Funktion wie die genannte Sequenz SEQ ID NO:4, oder
c) einer Aminosäuresequenz, die zu wenigstens 80% mit der Aminosäuresequenz SEQ ID NO:4 identisch ist und dieselbe Funktion wie die genannte Sequenz SEQ ID NO:4 hat, oder
d) einer Aminosäuresequenz, die zu wenigstens 80% mit der unter b) definierten Aminosäuresequenz SEQ ID NO:4 identisch ist und dieselbe Funktion wie die genannte Sequenz SEQ ID NO:4 hat,
wobei selbstverständlich gilt:
dass man unter "dieselbe Funktion wie die genannte Sequenz SEQ ID NO:4" eine *Legionella* pneumophila-Antigenfunktion versteht.

2. Anwendung nach Anspruch 1, außerdem anwendend:
a) ein Polypeptid der Aminosäuresequenz SEQ ID NO:2, (2A1-Protein), oder
b) ein einen Teil der Aminosäuresequenz SEQ ID NO:2 umfassendes Polypeptid mit der gleichen Funktion wie die genannte Sequenz SEQ ID NO:2, oder
c) ein Polypeptid mit einer Aminosäuresequenz, die zu wenigstens 80% mit der Aminosäuresequenz SEQ ID NO:2 identisch ist und die gleiche Funktion wie die genannte Sequenz SEQ ID NO:2 hat, oder
d) ein Polypeptid mit einer Aminosäuresequenz, die zu wenigstens 80% mit der unter b) definierten Sequenz identisch ist und dieselbe Funktion wie die genannte Sequenz SEQ ID NO:2 hat,
wobei selbstverständlich gilt:
dass man unter "die gleiche Funktion wie die genannte Sequenz SEQ ID NO:2" eine *Legionella* pneumophila-Antigenfunktion versteht.

3. Anwendung nach Anspruch 1 oder 2, **dadurch gekennzeichnet, dass** das Polypeptid (oder die Polypeptide) erzeugt wird (werden) durch Kultur einer Wirtszelle, einen Rekombinationsvektor umfassend, mit einem eingebauten Polynukleotid, codierend für das genannte (oder die genannten) Polypeptid(e) des Kulturmilieus, und durch Isolierung des (oder der) genannten Polypeptids (Polypeptide) des Kulturmilieus.

4. Anwendung nach Anspruch 3, wenn er vom Anspruch 1 abhängig ist, **dadurch gekennzeichnet, dass** das Polynukleotid die Sequenz SEQ ID NO:3 aufweist (codierendes Polynukleotid für das G5-Protein).

5. Anwendung nach Anspruch 3, wenn er vom Anspruch 2 abhängig ist, **dadurch gekennzeichnet, dass** das Polynukleotid die Sequenz SEQ ID NO:1 aufweist (codierendes Polynukleotid für das 2A1-Protein).

6. Anwendung zur *In-vitro*-Detektion der Präsenz infolge einer *Legionella pneumophila*-Infektion in biologischen Proben erzeugter Antikörper:
a) des Polynukleotids der Sequenz SEQ ID NO:3, oder
b) eines einen Teil der Sequenz SEQ ID NO:3 umfassenden Polynukleotids mit der gleichen Funktion wie die genannte Sequenz SEQ ID NO:3, oder
c) eines Polynukleotids mit einer Polynukleotidsequenz, die zu wenigstens 80% mit der Sequenz SEQ ID NO:3 identisch ist und die gleiche Funktion wie die genannte Sequenz SEQ ID NO:3 hat, oder
d) eines Polynukleotids mit einer Polynukleotidsequenz, die zu wenigstens 80% mit der unter b) definierten Sequenz identisch ist und die gleiche Funktion wie die genannte Sequenz SEQ ID NO:3 hat, oder
e) einer Polynukleotidsequenz, komplementär zu einer der unter a), b), c) oder d) definierten Polynukleotidsequenzen,
wobei selbstverständlich gilt:
dass man unter "die gleiche Funktion wie die genannte Sequenz SEQ ID NO:3" eine Codierfunktion eines *Legionella pneumophila*-Antigenpolypeptids versteht.

7. Anwendung nach Anspruch 6, außerdem anwendend:
a) ein Polynukleotid der Sequenz SEQ ID NO:1, oder
b) ein Polynukleotid mit einem Teil der Sequenz SEQ ID NO:1 mit der gleichen Funktion wie die genannte Sequenz SEQ ID NO:1, oder
c) ein Polynukleotid mit einer Polynukleotidsequenz, die zu wenigstens 80% mit der Polynukleotidsequenz SEQ ID NO:1 identisch ist und dieselbe Funktion wie die genannte Sequenz SEQ ID NO:1 hat, oder
d) ein Polynukleotid, eine Polynukleotidsequenz umfassend, die zu wenigstens 80% mit der unter b) definierten Sequenz identisch ist und dieselbe Funktion wie die genannte Sequenz SEQ ID NO:1 hat,
e) ein Polynukleotid, eine Polynukleotidsequenz umfassend, komplementär zu einer der unter a), b), c) oder d) definierten Polynukleotidsequenzen,
wobei selbstverständlich gilt:
dass man unter "die gleiche Funktion wie die genannte Sequenz SEQ ID NO:1" eine Codierfunktion eines *Legionella pneumophila*-Antigenpolypeptids versteht.
